# EUROPEAN PATENT APPLICATION

(11) **EP 2 292 257 A2**
(43) Date of publication of application: **09.03.2011**
(21) Application number: 09177043.8
(22) Date of filing: 13.07.2005
(51) Int. Cl.: A61K 38/48

(54) **Compositions and methods for dermatological wound healing**

(30) Priority: 13.07.2004 US 587057 P
(62) Divisional of application: 05759914.4
(71) Applicant: Mediwound, Ltd., Yavne 81227 (IL)
(72) Inventor: Toren, Amir, 43211, Ra'anana (IL); Rosenberg, Lior, 84965, Omer (IL); Gorecki, Marian, 64235, Tel Aviv (IL)
(74) Representative: Becker Kurig Straus

(57) **Abstract**

The invention relates to compositions for debriding damaged skin comprising at least one enzymatic debriding agent and at least one antiseptic compound comprising heavy metal ions. The present invention further relates to kit and methods for topical debridement of damaged skin, particularly of burned skin.

## Description

### FIELD OF THE INVENTION

The present invention relates to compositions for preparing a necrotic wound bed for healing, comprising at least one antiseptic compound comprising heavy metal ions and further comprising at least one enzymatic debriding agent. The present invention further relates to kits and methods for topical debridement of damaged skin.

### BACKGROUND OF THE INVENTION

Applications of enzyme technologies to pharmaceutical research and therapeutic uses are well known in the art. Numerous therapeutic enzymes have been approved through the years by the U.S. Food and Drug Administration for a variety of therapies including lysis of blood clots and treatment of damaged tissue by proteolytic and glycolytic enzymes.

Treatment of burned skin, termed eschar, in acute burn injuries requires removal of the eschar tissue and preparing the wound bed for the healing process. This wound bed preparation and cleaning process is known as debridement and is typically attempted by drug treatment or non-surgical manual methods, including use of debriding enzymes, that have been devised to avoid the blood loss and pain inherent in surgery. The choice of treatment greatly depends on the depth and location of the wound. Efficacious enzymatic debridement is advantageous over other debriding methods, as it may provide an answer to the Bum Induced Compartment Syndrome (BICS) as disclosed in WO 03/090598 of the inventor of the present invention. Another advantage of enzymatic debridement and moreover of non-surgical debridement is that they lacks the danger of exposing the already badly injured patient to any additional trauma related to the surgical pressure release (escharotomy) and yet provide an immediate and a fast resolution of the emergency medical conditions associated with bum injuries.

Hydrolytic enzymes derived from the pineapple plant that are useful for digestion, dissection and separation of non-viable, especially eschar tissue, from viable tissue are disclosed in U.S. Patent Nos. 4,197,291; 4,226,854; 4,307,081; 4,329,430 and 5,830,739 among others.

Much of the morbidity and mortality of bum patients is connected with increased infections within the susceptible and often contaminated burned tissue and its surroundings. If untreated, severe infections can lead to permanent damage to tissues and organs, or even to the patient's death. This condition requires urgent attention and resolution, sometimes as a component of the primary resuscitation of a burn victim. The consequent risk arising from violent infection, that is the result of contaminated eschar, is well documented (e.g. Hummel et al., J Trauma 1974, 14:572-9; Krizek et al., J Surg Res 1974, 17:219-27).

There is an unmet need for compositions and methods for debriding eschar tissue efficiently without affecting the healthy surrounding tissue while simultaneously preventing development of infections and alleviating the transient pain during the initial proteolysis period.

### SUMMARY OF THE INVENTION

The present invention provides a composition for preparing a necrotic wound bed by debriding devitalized tissue, the compositions comprising at least one enzymatic debriding agent and at least one antiseptic compound comprising heavy metal ions. Advantageously, the compositions of the invention may be combined extemporaneously from the individual components provided in the form of a kit. The invention further relates to methods for debriding a devitalized tissue.

The present invention is based in part on the unexpected discovery that the debriding activity of debriding enzymes, or of a composition comprising same, is maintained in the presence of an antiseptic compound comprising heavy metal ions. These findings are completely unexpected particularly in view of the association of heavy metal ions, especially silver ions, with attenuation or inhibition of enzymatic activity. Although inhibition of enzymatic activity by silver ions or other heavy metal ions is known to occur for specific enzymes or enzymatic complexes, it is now disclosed that surprisingly silver ions do not affect the activity of the compositions of the present invention in the time period required for debridement of a wound bed.

The present invention provides advantageous compositions for enzymatic wound debridement. The composition and methods of the present invention are particularly suitable for debridement of deep bums. Enzymatic wound debriding as known in the art yypically involves two stages of treatment: first, the wound site is cleansed thoroughly using antiseptic compositions and second, the enzymatic debriding composition is applied to the wound site with a time interval of about 2 to 24 hours between the two stages. This time interval between the cleansing step and the debriding step may lead to the development of severe infection. The present invention overcomes the risks that may arise as a result of delayed debridement, by providing compositions for enzymatic debridement comprising antiseptic components. Using the compositions of the present invention provides a one-step enzymatic debridement. Consequently, by using the compositions of the present invention the bacterial load at the wound site is reduced and moreover the tissues surrounding the wound site are protected from contamination by the released bacteria.

According to one aspect, the present invention provides a composition comprising at least one enzymatic debriding agent and at least one antiseptic compound comprising heavy metal ions.

According to one embodiment, the at least one enzymatic debriding agent comprises an enzyme of bacterial, plant or animal origin. In preferred embodiments the at least one enzymatic debriding agent comprises enzymes selected from the group consisting of: enzymes derived from the pineapple including but not limited to bromelain or debridase; trypsin; fibrinolysin or fibrinolysin with deoxyribonuclease; *Clostridium histolyticum* enzyme H-4; collagenase; *Bacillus subtilis* enzyme sutilains; *Streptococci* enzymes streptokinase or streptodornase; and derivatives of papaya including papain or papain-urea.

According to a preferred embodiment, the at least one enzymatic debriding agent comprises debridase.

According to yet another embodiment, the at least one antiseptic compound has at least one of the following antimicrobial activities: antifungal, antibacterial, antiviral and antiprotozoal.

According to yet another embodiment, the at least one antiseptic compound is selected from the group consisting of: silver sulphadiazine and silver ions compositions.

According to alternative embodiments the composition further comprises chemicals with debriding activity, for example acetic acid, pyruvic acid, phosphoric acid, salicylic acid, benzoic acid, malic acid.

According to yet another embodiment, the composition further comprises at least one anti-microbial substance selected from the group consisting of: broad-spectrum antibiotics, chlorohexidine, povidone iodine, mafenide acetate, neosporin, metronidazole, chlorine dioxide (ClO₂) and polymyxin B sulfate.

According to alternative embodiments the composition further comprises at least one analgesic agent. According to one embodiment, the at least one analgesic agent is selected from the group consisting of: lidocaine, tetracaine, procaine, mepivacaine, benzocaine, bupivacaine, prilocaine, etidocaine and a combination thereof.

According to another embodiment, the composition further comprises a pharmaceutically acceptable diluent or carrier. According to yet another embodiment, the pharmaceutically acceptable diluent or carrier comprises at least one substance selected from the group consisting of: water, organic solvent, inorganic solvent, buffering agent, acidifying agent and alkalizing agent. According to yet another embodiment, the pharmaceutically acceptable diluent or carrier may further comprise ointment bases, antimicrobial preservatives, antioxidants, plasticizers and stiffening agents.

According to yet another embodiment, the composition is provided in a form selected from the group consisting of: gel, cream, emulsion, foam, mousse, slurry, spray, paste, suspension and ointment.

According to another aspect, the present invention provides a kit for removing a devitalized tissue, the kit comprising separate components of:
(a) a first component comprising at least one enzymatic debriding agent; and
(b) a second component comprising at least one antiseptic compound comprising heavy metal ions.

According to one embodiment, the at least one enzymatic debriding agent comprises an enzyme of bacterial, plant or animal origin. In preferred embodiments the enzyme is selected from the group consisting of: enzymes derived from the pineapple including, but not limited to, bromelain or debridase; trypsin; fibrinolysin or fibrinolysin with deoxyribonuclease; *Clostridium histolyticum* enzyme H-4; collagenase; *Bacillus subtilis* enzyme sutilains; *Streptococci* enzymes streptokinase or streptodornase; and derivatives of papaya including papain or papain-urea.

According to a preferred embodiment, the at least one enzymatic debriding agent comprises debridase.

According to alternative embodiments the first component further comprises chemicals with debriding activity, for example acetic acid, pyruvic acid, phosphoric acid, salicylic acid, benzoic acid, malic acid.

According to another embodiment, the first component is provided in a storable non-active form. The storable non-active form of the first component may be frozen, or in the form of a lyophilizate, precipitate or crystal.

According to yet another embodiment, the second component has at least one of the following antimicrobial activities: antifungal, antibacterial and antiviral.

According to yet another embodiment, the at least one antiseptic compound is selected from the group consisting of: silver sulphadiazine, silver ions compositions, and heavy metal chlorites.

According to yet another embodiment, the second component further comprises at least one anti-microbial substance selected from the group consisting of: broad-spectrum antibiotics, chlorohexidine, povidone iodine, mafenide acetate, neosporin, metronidazole, chlorine dioxide (ClO₂) and polymyxin B sulfate.

According to yet another embodiment, the second component is provided in a form selected from the group consisting of: gel, cream, emulsion, foam, mousse, slurry, spray, paste, suspension and ointment.

According to another embodiment, the kit further comprises a third component comprising at least one pharmaceutically acceptable diluent or carrier suitable for extemporaneous reconstitution of the storable non-active form of the debriding composition to an active form.

According to yet another embodiment, the pharmaceutically acceptable diluent or carrier comprises at least one substance selected from the group consisting of: water, organic solvent, inorganic solvent, buffering agent, acidifying agent and alkalizing agent.

According to yet another embodiment, the pharmaceutically acceptable diluent or carrier may further comprise ointment bases, antimicrobial preservative, antioxidant, plasticizer, and stiffening agent.

According to alternative embodiments the kit further comprises at least one analgesic agent.

According to one embodiment, the at least one analgesic agent is selected from the group consisting of: prilocaine, lidocaine, tetracaine, procaine, mepivacaine, benzocaine, bupivacaine and etidocaine.

According to yet another embodiment, the kit further comprises covering means. The covering means includes, but is not limited to, gauze, absorbent pad, fibrous nets, fibrous knotted nets, non-woven dressing, sponges, films and occlusive sheets. The covering means is made of natural or synthetic, stable or degradable materials known in the art.

According to yet another embodiment, the kit further comprises mixing and spreading means for the reconstitution of the active debriding agent and its application on the desired target area, including but not limited to, a spatula, a hand-operated dispenser and a sponge.

The present invention is based in part on the unexpected discovery that treating bums with antiseptic agents comprising heavy metal ions, particularly silver ions, in conjunction with or followed immediately by application of debriding compositions, is efficacious. As exemplified hereinafter, according to the principles of the present invention use of debriding compositions which comprise enzymes derived from the pineapple, provides particularly efficient debridement of the burn eschar devoid of infection-related complications.

According to yet another aspect, the present invention provides a method for debridement of devitalized tissue comprising contacting a devitalized tissue with a composition comprising at least one enzymatic debriding agent and at least one antiseptic compound comprising heavy metal ions.

According to an alternative embodiment, the method comprising:
(a) providing an antiseptic composition comprising at least one antiseptic compound comprising heavy metal ions;
(b) providing a debriding composition comprising at least one enzymatic debriding agent;
(c) contacting a devitalized tissue with the antiseptic composition; and
(d) contacting, concomitantly with step (c), the devitalized tissue with the debriding composition.

According to yet another embodiment the method further comprising covering the said devitalized tissue.

According to one embodiment, the method of the invention further comprises the following steps:
(e) determining disintegration of said devitalized tissue; and
(f) removing said disintegrated devitalized tissue.

According to another embodiment, the present invention provides subsequent rounds of treatment in iteratively repeated steps, reapplying the method of the invention either in its entirety or in selected steps only.

According to yet another embodiment, said devitalized tissue is covered with sterile covering means.

According to yet another embodiment, the covering means is occlusive. According to an alternative embodiment the covering means is water-impermeable and is capable of retaining an aqueous solution or suspension. Additionally the covering may be elastic or pliant so as to accommodate itself to the contours of the organ that includes the damaged tissue.

According to yet another embodiment, the debriding composition is provided in a storable non-active form and is reconstituted in a pharmaceutically acceptable carrier or diluent prior to step (d).

Preferably, the debriding composition is reconstituted no longer than 60 minutes before step (d).

According to an alternative embodiment, the storable non-active debriding composition is reconstituted in the antiseptic composition.

Other objects, features and advantages of the present invention will become clear from the following description and drawings.

### BRIEF DESCRIPTION OF THE DRAWINGS

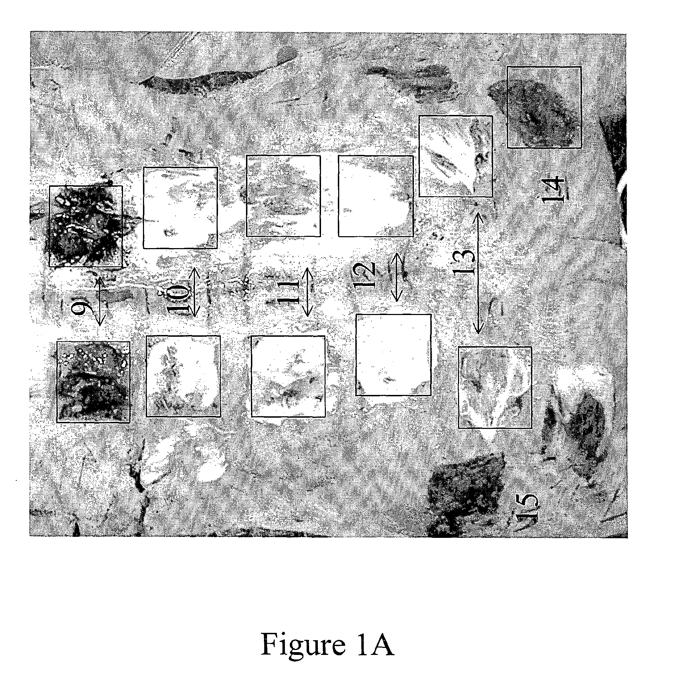

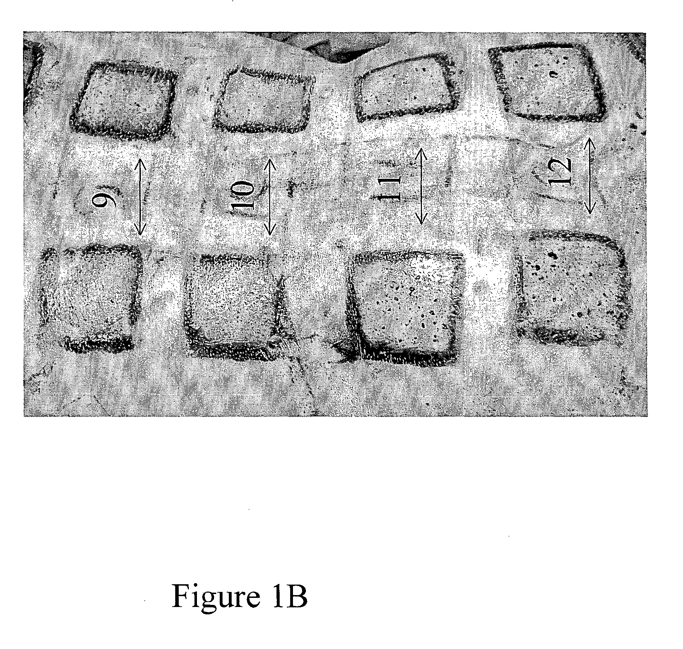

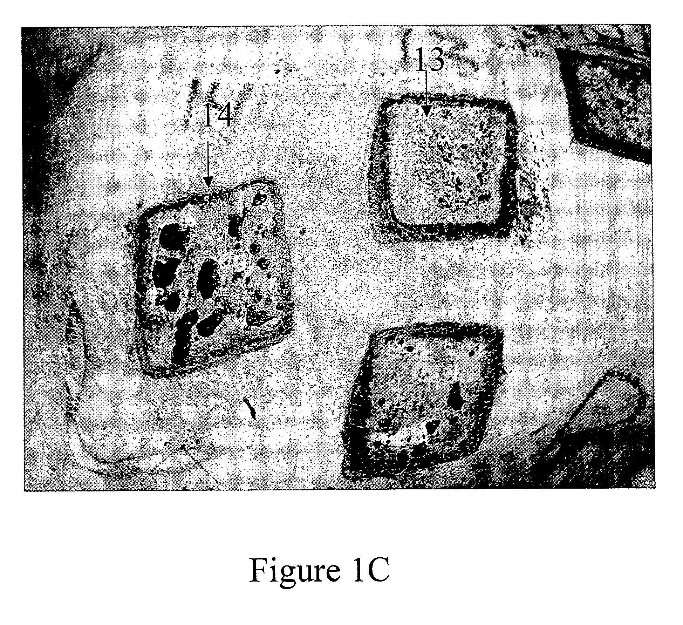

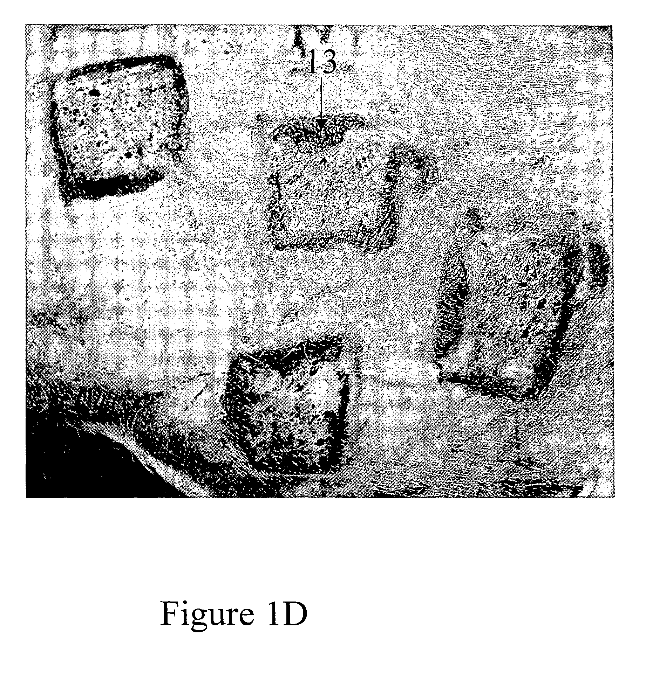

Figure 1 shows the debriding effects obtained using a composition comprising debriding enzymes reconstituted in antiseptic composition and hydrating gel *in vivo,* the antiseptic composition containing silver sulfadiazine (areas no. 10-13) or AgNO₃ (area nos. 14-15) with respect to control (area no. 9), after opening the occlusive dressing (A) and following cleaning the dissolved eschar (B-D).

### DETAILED DESCRIPTION OF THE INVENTION

The present invention relates to a composition for debriding devitalized tissue. The composition comprises an enzymatic debriding composition and an antiseptic composition comprising heavy metal ions. The composition preferably comprises an analgesic agent. The analgesic agent is preferably an agent optimal for local anesthesia, including but not limited to, at least one of the following: prilocaine, lidocaine, tetracaine, procaine, mepivacaine, benzocaine, bupivacaine and etidocaine or a combination thereof, for example, the eutectic mixture of lidocaine and prilocaine (EMLA^{®}).

The present invention further relates to a method for debridement of a devitalized tissue comprising contacting a damaged tissue, encompassing a devitalized tissue, with a debriding composition comprising debriding enzymes and an antiseptic composition comprising heavy metal ions. Following preparation of the necrotic wound bed using the compositions and methods of the present invention, the healing process is initiated either by epithelialization, by second intention (scar formation) or by skin grafting.

### Debriding compositions

The debriding composition according to the present invention comprises at least one enzymatic debriding agent of bacterial, plant or animal origin. In preferred embodiments the at least one enzymatic debriding agent is selected from the group consisting of: enzymes derived from the pineapple including but not limited to bromelain or debridase exemplified herein below; trypsin; fibrinolysin or fibrinolysin-deoxyribonuclease (such as the product known by the tradename ELASE^{®}); *Clostridium histolyticum* enzyme H-4; collagenase (such as the product known by the tradenames VARIDASE® or SENTYL®); *Bacillus subtilis* enzyme sutilains; *Streptococci* enzymes streptokinase or streptodornase; and derivatives of papaya including papain or papain-urea (such as that known by the tradenames ACCUZYME® and PANAFIL®).

According to a preferred embodiment, the at least one enzymatic debriding agent comprises debridase.

According to alternative embodiments it is also advantageous to include within the debriding composition chemicals with debriding activity, for example acetic acid, pyruvic acid, phosphoric acid, salicylic acid, benzoic acid, malic acid (such as the product known by the tradename ASERBIN®), urea or INTRASITE® gel (Smith and Nephew Ltd.).

Without being limited thereto, the composition of the invention is the form of suspension, gel, cream, lotion, emulsion, foam, mousse, slurry, spray, paste, drops or ointment.

Compositions according to the present invention may also comprise any conventional carrier or adjuvant used in pharmaceuticals, personal care formulations and compositions or veterinary formulations, as detailed hereinbelow.

The present invention further provides a kit comprising a storable form of debriding composition. The debriding composition is then provided in a solid form such as frozen form, crystallized or lyophilized dry form and is activated upon reconstitution in a suitable carrier or diluent or other "hydrating means" prior to use. According to a certain embodiment the storable debriding composition is provided in a lyophilizate powder. The dry debriding powder is activated in situ by the addition of an aqueous solution, such as physiological saline, purified water and any other suitable solution. In alternative embodiments the dry debriding composition powder further comprises excipients that will form a gel or emulsion or other viscous solution upon hydration with hydrating means. The excipients may include synthetic polymers, natural polymers or any combinations thereof as are well known in the art.

The term "storable form" refers to the solid physical form of the stored component having sufficiently long shelf life. In the case of a component having one or more particular activities, such as an enzyme, the storable form is substantially inactive. Activity can be regained by reconstituting the storable form of the component in a suitable hydrating means. The concentration of the active component in the reconstituted mixture or composition depends on the amount of activity that is desired.

Preferably, the kit of the invention further comprises pharmaceutically acceptable diluent or carrier suitable for reconstitution of the debriding composition. The reconstituting solution may further comprise excipients and/or auxiliaries.

Without being limited thereto, upon reconstitution the debriding composition is in a physical form of suspension, gel, cream, lotion, emulsion, foam, mousse, slurry, spray, paste, drops or ointment.

Optimally, the hydrating means will be used at 30°C-40°C. It is possible to use hydrating means at room temperature, though preferably the hydrating means, such as saline, will be preheated to approximately body temperature before reconstitution of the solid debriding composition.

Reconstitution of frozen, crystallized or lyophilized material has been shown to be effective with conventional proteins and peptides using reconstitution techniques as are known in the art. It will be appreciated by those skilled in the art that freezing, crystallization or lyophilization and reconstitution of these forms can lead to varying degrees of activity loss and that use levels may have to be adjusted to compensate.

Debriding compositions in the form of gels, creams or pastes or other viscous and mobile physical form are notably advantageous as they enable an intimate physical contact with the irregular surface of a wound, something that is often not achieved with rigid or substantially fluid physical forms such as powders or solutions . The quality of the contact is however tempered by the conflicting needs of making the gel, cream or paste sufficiently mobile that they can be applied to the wound but not so mobile that it runs out of the wound under the influence of gravity. Debriding composition in the form of a gel, cream or paste are usually applied by squeezing the composition from a tube or other suitable container by hand, optionally using sterile spatula, wooden tongue depressor, or other suitable means. Another significant advantage of such forms are their inherent humidity which may also assist in preventing the wound from drying out, thereby promoting healing.

The amount of debriding substance within the debriding composition may vary depending upon the therapeutic dosage recommended or permitted for the particular debriding substance. In general, the amount of debriding substance present in the composition is the ordinary dosage required to obtain the desired therapeutic result.

The terms "therapeutic dosage" or "pharmaceutically effective amount" as used herein refer to an amount of a debriding substance, debriding composition or antiseptic formulation or composition that provides an effective treatment within a defined area of eschar over a define time interval.

Debriding compositions according to the present invention may also comprise any conventional carrier or adjuvant used in pharmaceuticals, personal care formulations and compositions or veterinary formulations. These carriers and adjuvants include, the following:
(i) Acidifying agents, such as, acetic acid, glacial acetic acid, citric acid, fumaric acid, hydrochloric acid, diluted hydrochloric acid, malic acid, nitric acid, phosphoric acid, diluted phosphoric acid, sulfuric acid and tartaric acid.
(ii) Alcohol denaturants, such as, denatonium benzoate, methyl isobutyl ketone and sucrose octacetate.
(iii) Alkalizing agents including strong ammonia solution, ammonium carbonate, diethanolamine, diisopropanolamine, potassium hydroxide, sodium bicarbonate, sodium borate, sodium carbonate, sodium hydroxide or trolamine.
(iv) Antifoaming agents, e.g. dimethicone and simethicone.
(v) Antimicrobial preservatives, such as, benzalkonium chloride, benzalkonium chloride solution, benzelthonium chloride, benzoic acid, benzyl alcohol, butylparaben, acetylpyridinium chloride, chlorobutanol, chlorocresol, cresol, dehydroacetic acid, ethylparaben, methylparaben, methylparaben sodium, phenol, phenylethyl alcohol, phenylmercuric acetate, phenylmercuric nitrate, potassium benzoate, potassium sorbate, propylparaben, propylparaben sodium, sodium benzoate, sodium dehydroacetate, sodium propionate, sorbic acid, thimerosal and thymol.
(vi) Antioxidants, such as, ascorbic acid, ascorbyl palmitate, butylated hydroxyanisole, butylated hydroxytoluene, hypophosphorous acid, monothioglycerol, propyl gallate, sodium formaldehyde sulfoxylate, sodium metabisulfite, sodium thiosulfate, sulfur dioxide, tocopherol and tocopherols excipient.
(vii) Buffering Agents, including acetic acid, ammonium carbonate, ammonium phosphate, boric acid, citric acid, lactic acid, phosphoric acid, potassium citrate, potassium metaphosphate, potassium phosphate monobasic, sodium acetate, sodium citrate, sodium lactate solution, dibasic sodium phosphate and monobasic sodium phosphate.
(viii) Ointment Bases, including lanolin, anhydrous lanolin, hydrophilic ointment, white ointment, yellow ointment, polyethylene glycol ointment, petrolatum, hydrophilic petrolatum, white petrolatum, rose water ointment and squalane.
(ix) Plasticizers, e.g. castor oil, diacetylated monoglycerides, diethyl phthalate, glycerin, mono- and di-acetylated monoglycerides, polyethylene glycol, propylene glycol, triacetin and triethyl citrate.
(x) Solvents, for example, acetone, alcohol, diluted alcohol, amylene hydrate, benzyl benzoate, butyl alcohol, carbon tetrachloride, chloroform, corn oil, cottonseed oil, ethyl acetate, glycerin, hexylene glycol, isopropyl alcohol, methyl alcohol, methylene chloride, methyl isobutyl ketone, mineral oil, peanut oil, polyethylene glycol, propylene carbonate, propylene glycol, sesame oil, water for injection, sterile water for injection, sterile water for irrigation and purified water.
(xi) Sorbents, such as, powdered cellulose, charcoal, purified siliceous earth or carbon dioxide sorbents (e.g. barium hydroxide lime, soda lime).
(xii) Stiffening Agents, for example, hydrogenated castor oil, cetostearyl alcohol, cetyl alcohol, cetyl esters wax, hard fat, paraffin, polyethylene excipient, stearyl alcohol, emulsifying wax, white wax and yellow wax.
(xiii) Agents that promote penetration into the eschar, e.g., urea, non-ionic detergents e.g. NP-40, Triton X-100, ionic detergents such as sodium dodecyl sulphate and lauryl dodecyl sulphate.

### Antiseptic compositions

The term "antiseptic" as used herein is in accordance with the meaning normally assigned thereto in the art and further described herein. An antiseptic agent or composition having an antimicrobial activity comprising one or more of the following activities: antifungal activity, antibacterial activity, antiviral activity and/or antiprotozoal activity.

The particular antiseptic and antibiotic agents of the antiseptic composition according to the present invention are selected from those commonly known and available in the medical industry with the proviso that the antiseptic composition comprises heavy metal ions. Other agents, such as anesthetics, can be also included within the antiseptic composition.

Typical antiseptic compositions that enable the working of the present invention are disclosed in U.S. Patent No. 5,384,134; 5,652,274; and 5,855,922 among others.

According to one embodiment, the antiseptic composition comprises at least one anti-microbial substance selected from the group consisting of: silver sulphadiazine (also known by the tradenames SILVADINE® and SILVEROL®), AgNO₃, silver ions compositions, and heavy metal chlorites.

According to yet another embodiment, the antiseptic composition further comprises at least one anti-microbial substance selected from the group consisting of: broad-spectrum antibiotics, chlorohexidine, povidone iodine, mafenide acetate (e.g. SULFAMYALON^{®}), neosporin, metronidazole, chlorine dioxide (ClO₂) and polymyxin B sulfate.

The antiseptic composition may further comprise steroidal anti-inflammatory agents (e.g. corticosteroids and synthetic analogs thereof) and antifungal agents such as nystatin and econazole nitrate.

Silver sulphadiazine (also known as Silvadene™, Thermazene™, Sylverol® and Argent-Eze® among others) has antibacterial activity especially against Gram-negative organisms such as *Pseudomonas aeruginosa* and *pyocyanea*, a common cause of burn wound infection. Silver sulphadiazine is commonly used for topical prevention of infection in severe bums.

The antibacterial activity of silver sulphadiazine is achieved through the formation of sustained release depot of silver and sulphadiazine at the wound surface. As silver sulphadiazine is relatively insoluble it reacts very slowly with the chloride and protein components of tissue exudate to form silver chloride, silver protein complexes and sodium sulphadiazine. The slow liberation of silver does not cause the rapid and extensive depletion of chloride ion experienced when silver nitrate solutions are used, and thus electrolyte disturbances are minimized. Generally the mechanisms for silver ion release are complex, but silver chloride is slightly soluble and slowly releases silver ions, which are then free to exert their bactericidal effect. These silver ions are thought to be reversibly adsorbed by bacterial cells by association with SH groups or histidine residues in the bacterial protein of the transport system across the cell wall. Silver based antiseptic compositions as known in the art are typically used in a form of solution (e.g. AgNO₃ solution), dressings (e.g. Acticoat™ by Smith & Nephew and AgAcquacell™ by Convatec) or topical preparations such as Silvadene^{®} cream (silver sulphadiazine) by Monarch. These and similar preparations could be used in conjuncture with the enzymatic debriding agent of the invention.

It should be noted that the texture of the combination containing the antiseptic and the debriding compositions might be different from the texture of each component by itself. A typical example is the transformation of the debriding agent after reconstitution with hydrating gel vehicle from a viscous gel-like texture to a semi solid cake upon the addition of AgNO₃ (0.5%). This cake adheres to the treated area and performs debridement thereof (Figure 1). It has been found that use of an effective amount of a semi solid combination comprising antiseptic and debriding compositions is advantageous over use of a cream for application over difficult body areas such as folds, breasts, shoulders and head.

The antiseptic composition according to the present invention may further comprise antiviral agents selected from a wide variety of water-soluble and water-insoluble drugs and their acid addition or metallic salts. Both organic and inorganic salts may be used provided the antiviral agent maintains its medicament value. The antiviral agents may be selected from a wide range of therapeutic agents and mixtures of therapeutic agents which may be administered in sustained release or prolonged action form. Non-limiting illustrative categories of such antiviral agents include RNA synthesis inhibitors, protein synthesis inhibitors, immunostimulating agents, protease inhibitors, and cytokines. Nonlimiting illustrative specific examples of such antiviral agents include the following medicaments.
(i) Ribavirin (1-β-D-ribofuranosyl-1,2,4-triazole-3-carboxamide, trade name-Virazole™) is an antiviral drug provided as a sterile, lyophilized powder. The empirical formula is C₈H₁₂N₄O₅ and the molecular weight is 244.2 Daltons. Ribavirin has antiviral inhibitory activity in vitro against respiratory syncytial virus, influenza virus, and herpes simplex virus. Ribavirin is also active against respiratory syncytial virus (RSV) in experimentally infected cotton rats. In cell cultures, the inhibitory activity of ribavirin for RSV is selective.
(ii) Vidarabine (adenine arabinoside, Ara-A, 9-β-D-arabinofuranosyladenine monohydrate, trade name-ViraA™) is an antiviral drug. Vidarabine is a purine nucleoside obtained from fermentation cultures of Streptomyces with the empirical formula, C₁₀H₁₃N₅O₄·H₂O. Vidarabine possesses in vitro and in vivo antiviral activity against Herpes simplex virus types 1 and 2 (HSV-1 and HSV-2), and in vitro activity against varicella-zoster virus (VZV). Vidarabine is converted into nucleotides which inhibit viral DNA polymerase.
(iii) Phenol (carbolic acid, C₆H₆O) is a topical antiviral, anesthetic, antiseptic, and antipruritic drug.
(iv) Amantadine hydrochloride (1-adamantanamine hydrochloride, trade name-Symmetrel™) has pharmacological actions as both an anti-Parkinson and an antiviral drug. The antiviral activity of amantadine hydrochloride against influenza A appears to be the prevention of the release of infectious viral nucleic acid into the host cell.

The antiseptic composition according to the present invention may be used in many distinct physical forms well known in the pharmaceutical art to provide an effective antiseptic activity. Such physical forms include time-release forms of the antiseptic composition. Preferred physical forms include liquids, gel, cream, emulsion, foam, mousse, slurry, spray, paste, lyophilizate powder, suspension and ointment. The antiseptic compositions may be provided as films or dressing coated with same.

The amount of each antiseptic agent used in the antiseptic composition may vary depending upon the therapeutic dosage recommended or permitted for the particular agent. In general, the amount of antiseptic agent present is the ordinary dosage required to obtain the desired result. Such dosages are known to the skilled practitioner in the medical arts and are not a part of the present invention

### Methods of debridment

The present invention provides methods for debriding devitalized tissue in a wound comprising applying enzymatic preparation in conjunction with antiseptic compositions.

According to one embodiment, the present invention provides a method for debridement of devitalized tissue comprising:
(a) contacting a devitalized tissue with at least one antiseptic compound;
(b) contacting, concomitantly with step (a), the devitalized tissue with debriding composition comprising at least one enzymatic agent; and, optionally,
(c) covering said devitalized tissue.

According to one embodiment, the method of the invention further comprises removing said devitalized tissue.

The terms "concomitantly" or "concomitant administration" are interchangeably used herein and refer to a simultaneous application or application substantially at the same time.

According to another embodiment, the method further comprises contacting the devitalized tissue with hydrating means prior to step (b). The hydrating means may comprise topical anesthetic and antiseptic gents. Hydrating means that provide hydration together with analgesic action may comprise at least one compound selected from the group consisting of: prilocaine, lidocaine, tetracaine, procaine, mepivacaine, benzocaine, bupivacaine and etidocaine or a combination thereof, such as, the eutectic mixture of lidocaine and prilocaine (EMLA^{®}), a mixture of benzocaine and lidocaine (ZILACTIN^{®}).

According to another embodiment, the present invention provides subsequent rounds of treatment in iteratively repeated steps, reapplying the method of the invention either in its entirety or in selected steps only.

According to yet another embodiment, said devitalized tissue is covered with sterile covering means as exemplified hereinafter.

According to yet another embodiment, the present invention provides a method for debridement of devitalized tissue comprising:
(a) providing a storable non-active debriding composition and an antiseptic composition;
(b) reconstituting the storable debriding composition in a pharmaceutically acceptable solution to obtain an active debriding composition.
(c) contacting a devitalized tissue with the antiseptic composition;
(d) contacting, concomitantly with step (c), the devitalized tissue with the active debriding composition; and, optionally,
(e) covering said devitalized tissue.

Preferably, the debriding composition is reconstituted no longer than 60 minutes before contacting same with the devitalized tissue.

According to an alternative embodiment, the method further comprises contacting the devitalized tissue with hydrating means prior to step (b). The hydrating means may comprise topical anesthetic and antiseptic agents. The active debriding composition may be prepared by mixing or suspending a dry or frozen active debriding composition with excipients or ingredients such as sugars, sugar alcohols, viscosity increasing agents, wetting agents, diluent, carriers and enhancer, such as, surfactant or solubilizing agents, buffer salts, emulsifying agents, antimicrobial agents, antioxidants and stabilizers.
According to an alternative embodiment, the storable non-active debriding composition is reconstituted in the antiseptic composition. Preferably, the antiseptic composition comprises water.

The interval between dressing changes will depend entirely upon the state of the wound. On heavily exuding or malodorous wounds frequent changes, such as at least once a day, are required but on dry wounds the dressing may be changed less often, for example, on alternate days. It is recommended that the dressing is not left *in-situ* for longer than three days between changes without a thorough clinical assessment. At the discretion of the medical officer in charge, treatment of wounds that show evidence of clinical infection may require the addition of appropriate systemic antibiotic therapy.

The raw surface that is left after debridement, also known as the "interface layer" comprises the upper layer of the healthy tissue. In all cases where any dermal elements survive the original trauma and the debridement process, the interface layer preserves all viable components such as epithelial elements and dermal remnants that are the basis of spontaneous epithelialization and healing. Preferably, the interface layer and the viable elements thereof are protected, mainly from desiccation, by providing adequate epithelialization conditions, such as moisture, which enhance healing (epithelialization). In the cases of a deep damage (such as third degree bums) or after the spontaneous healing potential has been exhausted grafting is required. A graftable bed may be obtained by a simple exposure of the blood vessels. Exposure may be achieved by a simple scrubbing of the Interface Layer.

### Wound dressing

The kit of the present invention may comprise dressing means intended for covering the damaged skin and its surroundings before, during and after the debriding treatment.

Before the debriding treatment the goal of the dressing is to assist hydration and topical anesthesia and prevent contamination. During the debriding treatment the dressing, preferably occlusive, is applied in order to maintain the antiseptic and the debriding compositions in place and provide the proper functional environment. After the debriding treatment the dressing means is topically applied and is aimed to assist in attenuating the formation of microbial infection, speeding wound healing and reducing skin damage.

The terms "occlusive" or "occlusive dressing" are interchangeably used herein to describe dressing that surrounds or covers a damaged area and may also surrounds or covers healthy tissue in the periphery of the damaged area. This dressing does not allow leakage of material from (such as exudate) or out of the area surrounded or covered by the dressing.

Covering means may be in the form of fibrous nets, fibrous knotted nets, gauze, non-wovens, sponges or honeycomb absorbent pad, perforated film absorbent dressing, such as Melolin™ (Smith & Nephew Healthcare Ltd.), Telfa™ (The Kendall Company Ltd.) or any other acceptable form and may be made of natural or synthetic, stable or degradable material, as those described in "Remington's Pharmaceutical Sciences", Mack Publishing Company, 1990, pages 1895-1900 or other similar source manuscripts.

The covering means may be water-impermeable thereby rendering it capable of retaining an aqueous solution or suspension. Additionally the covering may be elastic or pliant so as to accommodate itself to the contours of the organ that includes the damaged tissue.

The selection of the cover dressing is governed by the condition of the wound. If significant quantities of exudate are anticipated, a simple absorbent pad may be used, held in position with tape or a bandage, as appropriate. On lightly exuding wounds, a less permeable secondary dressing may be required, such as a perforated film absorbent dressing (Melolin or Telfa, for example). If the wound is very dry, a more occlusive covering may be used to reduce water vapor loss and to prevent the dressing drying out, a film dressing such as Opsite Flexigrid is suitable for this purpose. In the management of difficult wounds such as the hand or foot, the dressing may be retained on the wound in a suitably shaped plastic bag, forming a simple glove or boot.

The antiseptic composition together with the debriding composition may also be incorporated into a variety of material to produce wound dressings, compresses, and covers with surface and antimicrobial activity for direct topical application to the dermal surface. Examples of material used for wound dressings which are suitable for incorporation of the compositions of the invention include, but are not limited to: water-permeable, flexible, porous, sponge-type material; collagen sheets; Hyluronic acid preparations, composite dressings (collagen or Hyaluronic acid and synthetic products, composite collagen and Hyaluronic acid), hydrophilic polymer materials with a water content of from about 10 percent to about 90 percent; open cell foam type materials of natural and artificial rubber and urethane foams; and woven or non-woven fabrics of natural or synthetic materials. The wound dressings may be used together with the debriding composition or as a healing-enhancing cover on the clean interface layer.

The following examples are to be construed in a non-limitative fashion and are intended merely to be illustrative of the principles of the invention disclosed.

### EXAMPLES

### Materials and Methods

The debriding mixture was purified from Bromelain SP (raw material) which is a mixture of lyophilized proteolytic enzymes extracted from the stem of the pineapple plant. The resulting enzymatic debriding mixture is capable of rapid removal of bum eschar within 2 to 4 hours, leaving, in most cases, a wound bed devoid of necrotic tissue and suitable for further treatment by grafting or conservative methods. The debriding activity is specific, selective and limited to devitalized tissue only.

Purification process of the debriding mixture from bromelain involves protein purification including extraction with reducing agent, ammonium sulfate precipitation, and dissolution, removal of insoluble matters and low molecular substances and finally concentration. The preparation is freeze-dried and stored in the form of a homogenous dry powder.

Debriding composition was obtained by mixing two components which are stored in separate packing until use: (i) the debriding mixture powder and (ii) carbomer hydrating gel (such as Carbomer 940 and 980, Hyaluronic acid and dextrane).

**Table 1 - A typical gel formulation**

| Compound | %(W/W) | g/20g | g/50g |
|---|---|---|---|
| Carbomer 980*, NF | 2.0 | 0.40 | 1.00 |
| di-Sodium hydrogen phosphate, USP | 2.7 | 0.54 | 1.35 |
| Chlorocresol (4-chloro-3-methylphenol) | 0.10 | 0.02 | 0.05 |
| Purified water, USP | 95.2 | 19.04 | 47.60 |
| Sodium hydroxide 5N added as needed for a final pH of 7.4 | | | |

| | | | |
|---|---|---|---|
| *For example, Carbopol 980 by Goodrich (USA) | | | |

This study assessed the eschar-debriding activity of the debriding composition *in vivo* using an animal model of young domestic pig (Landrace x Large Whites juvenile swine) 22 Kg ± 2 Kg, bearing deep thermal burn. This model is well established for cutaneous thermal bums, as it is simulating appropriately a similar situation in humans. In order to create test conditions bums were inflicted in fully anesthetized animals, in a dorsal test area, by a radiant heat device creating wounds of about 4.5x4.5 cm². The treatment consisted of a 4-hour application of the debriding composition following by cleansing of the wound. Visual evaluation of the wound and extent of debridement is carried out by photographical documentation of the bum was performed before and after the debriding treatment. The results were expressed using debridement visual assessment scoring (VAS) scale of 1 to 5 as follows:

**Table 2 - VAS scoring**

| **VAS score** | **Description** |
|---|---|
| 0 | No effect |
| 1 | Small amount of eschar removed |
| 2 | 50% of the original eschar mass removed. Thrombosed dermal vessels may be visible |
| 3 | Most eschar removed, down to shiny surface |
| 4 | All eschar removed. Some bleeding points may be seen on the underlying dermis/fat, some islands of eschar remnants may be seen but not covering more than 33% of the wound surface |
| 5 | All eschar removed. Some bleeding points may be seen on the underlying dermis/fat. |

### Example 1 - Stability of the debriding composition

The activity, proteolytic and amidolytic, of the debriding mixture was investigated by an animal assay for eschar removal in young swine model. The results indicated that the activity of the debriding mixture following 3 or 9 months of storage at 25°C, 2-8°C and -18°C (Table 3, locations 3-6 and 9-12) remains similar to the activity of fresh mixtures (Table 3, locations 1 and 2).

### Example 2 - Debridment with the debriding composition and silver sulphadiazine or AgNO₃

The model was a radiant-heat thermal bum in an anesthetized young domestic pig. In order to create test conditions which are in accordance with the therapeutic use of the test article, deep dermal and full thickness burns were inflicted in all animals by applying a radiant heat device to the back to cover an area of approximately 3% TBSA (4.5 x 4.5 cm) for each burn. A gross evaluation of the debriding activity of the test article (the amount of eschar removed) was assessed and photographically documented. According to the dosage assay, the most effective final dosage was 2g of the debriding mixture powder per100 cm² bum, was mixed with one of the following (a) 20g of hydrating gel; (b) Silverol™;(c) hydrating gel with AgNO₃ 0.5%; (d) AgNO₃ 0.5% with di-sodium hydrogen phosphate USP 2.7% W/W in water.

Silverol™ did not attenuate and did not inhibit the debriding activity of the debriding mixture relative to the activity observed in the other locations. In locations 7 left and 7 right Silverol™ was used as a hydrating gel. The results indicate that Silverol™ does not interfere with the debriding activity of the debriding mixture. The activity of the debriding composition in the presence of Silverol™ was more effective than the activity of this composition in the presence of mafenide acetate, an (alpha)-amino-(rho)-toluenesulfonamide monoacetate (also known as Sulfamylon^{®}) which is a antimicrobial agent for topical applications (Table 3, locations 7 and 8, and respectively). Thus, using compositions comprising heavy metal ions, particularly silver ions, together with the debriding composition comprising enzymes is advantageous over the use of debriding composition having enzymes together with standard antiseptics devoid of heavy metal ions. This finding is particularly unexpected in view of the well known inhibitory activity that silver ions exert on enzymatic preparations.

All wounds were treated conservatively, post-debridement, with silver sulfadiazine (SSD) for the duration of the study (two weeks).

A gross evaluation of the debriding activity of the thermal bums in vivo (the amount of eschar removed) was also photographically documented (Fig. 1A-D).

Bums were treated with the debriding composition comprising the following components: silver sulfadiazine (Fig. 1 - areas no. 10-13), AgNO₃ (Fig. 1 - areas no. 14-15) and standard hydrating vehicle devoid of antiseptics (control, Fig. 1 - area no. 9). After applying the antiseptic debriding mixture onto the burned skin, the burned skin was occlude with an occlusive dressing as disclosed for example in European Patent No. 1014905.

Following cleaning of the dissolved eschar, the morphology of the debrided burns was observed (Fig. 1 B-D). Figure 1B presents the left and the right regions of locations 9-12. Figures 1C and 1D correspond to the right and left regions of several locations, respectively. No significant difference was observed between the areas that were treated with a debriding mixture comprising Silverol™ or AgNO₃ (areas 10-12 and 14-15, respectively). The most effective dose was 1-2 g of the debriding mixture powder mixed with Silverol™ for 100 cm² burn

**Table 3 - In vivo eschar debridement with a debriding mixture (MWD03) test in Swine Model.**

| **Location on limb** | **Sample lot** | **Quantity** **(gr)** | **Results** **(Left limb)** | **Results** **(Right limb)** | **Final** **Result** |
|---|---|---|---|---|---|
| 1 | MWD | 5 | 5 | 5 | 5 |
| 2 | MWD | 5 | 4 | 4 | 4 |
| 3 | MWD T3/25°C | 5 | 4.5 | 4.5 | 4.5 |
| 4 | MWD T9/2-8°C | 5 | 4.5 | 4 | 4.25 |
| 5 | MWD T9/-18°C | 5 | 4.5 | 4 | 4.25 |
| 6 | MWD W2/25°C | 4 | 6 | 4 | 4 |
| 7 | MWD + silver sulphadia zine | 5 | 4.5 | 3.5-4 | 4 |
| 8 | MWD + mafenide acetate | 5 | 3 | 3.5 | 3.25 |
| 9 | MWD T1/25°C | 0.5 | 4 | 4 | 4 |
| 10 | MWD T1/25°C | 0.5 | 4 | 4 | 4 |
| 11 | MWD T6/2-8°C | 5 | 5 | 5 | 5 |
| 12 | MWD Stability T6/2-8°C | 5 | 5 | 4.5 | 4.75 |

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

## Claims

1. A composition comprising at least one enzymatic debriding agent and at least one antiseptic compound comprising heavy metal ions.

2. The composition according to claim 1, wherein the at least one enzymatic debriding agent comprises enzymes derived from an organism selected from a group consisting of: bacteria, plant and animal.

3. The composition according to claim 2, wherein the at least one enzymatic debriding agent comprises enzymes derived from pineapple.

4. The composition according to claim 1, wherein the at least one enzymatic debriding agent comprises enzymes selected from a group consisting of: bromelain, debridase, trypsin, fibrinolysin, fibrinolysin-deoxyribonuclease, *Clostridium histolyticum* enzyme H-4, collagenase, *Bacillus subtilis* enzyme sutilains, streptokinase, streptodornase and papain.

5. The composition according to claim 1, further comprising at least one non-enzymatic agent with debriding activity.

6. The composition according to claim 5, wherein the at least one non-enzymatic agent is selected from a group consisting of: acetic acid, pyruvic acid, phosphoric acid, salicylic acid, benzoic acid, urea and malic acid.

7. The composition according to claim 1, wherein the at least one antiseptic compound has one or more of the following antimicrobial activities: antifungal, antibacterial, and antiviral.

8. The composition according to claim 1, wherein the at least one antiseptic compound comprises silver ions.

9. The composition according to claim 1, wherein the at least one antiseptic compound is selected from the group consisting of: silver sulphadiazine, AgNO₃ and metal chlorite.

10. The composition according to claim 1, further comprising at least one antimicrobial substance selected from the group consisting of: broad-spectrum antibiotics, chlorohexidine, povidone iodine, Sulfamyalone, neosporin, metronidazole, chlorine dioxide (ClO₂) and polymyxin B sulfate.

11. The composition according to claim 1, further comprising at least one analgesic agent.

12. The composition according to claim 11, wherein the at least one analgesic agent is selected from the group consisting of: prilocaine, lidocaine, tetracaine, procaine, mepivacaine, benzocaine, bupivacaine, etidocaine and a combination thereof.

13. The composition according to claim 1, further comprising a pharmaceutically acceptable diluent or carrier.

14. The composition according to claim 13, wherein the pharmaceutically acceptable diluent or carrier comprises at least one substance selected from the group consisting of: water, organic solvent, inorganic solvent, buffering agent, acidifying agent and alkalizing agent.

15. The composition according to claim 13, wherein the pharmaceutically acceptable diluent or carrier further comprises an ingredient selected from the group consisting of: ointment base, antimicrobial preservative, antioxidant, plasticizer and stiffening agent.

16. The composition according to claim 1, having a form selected from the group consisting of: liquids, gel, cream, emulsion, foam, mousse, slurry, spray, paste, suspension and ointment.

17. A kit for removing a devitalized tissue, the kit comprising separate components of:
(a) a first component comprising at least one enzymatic debriding agent; and
(b) a second component comprising at least one antiseptic compound comprising heavy metal ions.

18. The kit according to claim 17, wherein the at least one enzymatic debriding agent is derived from an organism selected from the group consisting of: bacteria, plant and animal.

19. The kit according to claim 18, wherein the at least one enzymatic debriding agent is derived from pineapple.

20. The kit according to claim 17, wherein the at least one enzymatic debriding agent is selected from the group consisting of: bromelain, debridase, trypsin, fibrinolysin, fibrinolysin-deoxyribonuclease, *Clostridium histolyticum* enzyme H-4, collagenase, *Bacillus subtilis* enzyme sutilains, streptokinase, streptodornase and papain.

21. The kit according to claim 17, wherein the first component further comprises non-enzymatic agents with debriding activity.

22. The kit according to claim 21, wherein the non-enzymatic agents are selected from a group consisting of: acetic acid, pyruvic acid, phosphoric acid, salicylic acid, benzoic acid, urea and malic acid.

23. The kit according to claim 17, wherein the first component is provided in a storable non-active form.

24. The kit according to claim 23, further comprising a third component comprising at least one pharmaceutically acceptable diluent or carrier suitable for reconstituting the first component to an active form.

25. The kit according to claim 24, wherein the pharmaceutically acceptable diluent or carrier comprises at least one substance selected from the group consisting of: water, organic solvent, inorganic solvent, buffering agent, acidifying agent and alkalizing agent.

26. The kit according to claim 24, wherein the pharmaceutically acceptable diluent or carrier further comprises an ingredient selected from the group consisting of: ointment base, antimicrobial preservative, antioxidant, plasticizer and stiffening agent.

27. The kit according to claim 17, wherein the at least one antiseptic compound has one or more of the following activities: antifungal, antibacterial and antiviral.

28. The kit according to claim 17, wherein the at least one antiseptic compound comprises silver ions.

29. The kit according to claim 17, wherein the at least one antiseptic compound is selected from the group consisting of: silver sulphadiazine, AgNO₃ and metal chlorite.

30. The kit according to claim 17, wherein the second component further comprises at least one substance selected from the group consisting of: broad-spectrum antibiotics, chlorohexidine, povidone iodine, Sulfamyalone, neosporin, metronidazole, chlorine dioxide (ClO₂) and polymyxin B sulfate.

31. The kit according to claim 17, wherein the second component is provided in a form selected from the group consisting of: liquids, gel, cream, emulsion, foam, mousse, slurry, spray, paste, suspension and ointment.

32. The kit according to claim 17, further comprising at least one analgesic agent.

33. The kit according to claim 32, wherein the analgesic agent is selected from the group consisting of: prilocaine, lidocaine, tetracaine, procaine, mepivacaine, benzocaine, bupivacaine, etidocaine and a combination thereof.

34. The kit according to claim 17, further comprising covering means.

35. The kit according to claim 34, wherein the covering means is selected from the group consisting of: films, sheets, gauze, absorbent pad, fibrous nets, fibrous knotted nets, non-woven dressing, sponges, mono or multi-layered sponges and composite materials.

36. The kit according to claim 17, further comprising topical medications for pre-debridement treatment and post-debridement treatment.

37. The kit according to claim 36, wherein the topical medications are selected from: hydrating substances and topical anesthetic means.

38. A method for enzymatic debridement of devitalized tissue, comprising:
(a) providing a composition comprising at least one enzymatic debriding agent and at least one antiseptic compound comprising heavy metal ions;
(b) contacting a devitalized tissue with the composition; and, optionally,
(c) covering the devitalized tissue with covering means.

39. The method of claim 39, wherein the antiseptic composition and the debriding composition are provided separately.

40. The method according to claim 38, further comprising removing said devitalized tissue.

41. The method according to claim 40, wherein the method is subsequently repeated in its entirety or in selected steps.

42. The method according to claim 38, wherein the at least one enzymatic debriding agent comprises enzymes derived from an organism selected from the group consisting of: bacteria, plant and animal.

43. The method according to claim 42, wherein the at least one enzymatic debriding agent comprises enzymes derived from pineapple.

44. The method according to claim 42, wherein the at least one enzymatic debriding agent comprises enzymes selected from the group consisting of: bromelain, debridase, trypsin, fibrinolysin, fibrinolysin-deoxyribonuclease, *Clostridium histolyticum* enzyme H-4, collagenase, *Bacillus subtilis* enzyme sutilains; streptokinase; streptodornase; papain and papain-urea.

45. The method according to claim 38, wherein the composition further comprises at least one non-enzymatic agent with debriding activity.

46. The method according to claim 39, wherein the debriding composition and the antiseptic compositions are provided together or consecutively onto the devitalized tissue.

47. The method according to claim 45, wherein the at least one non-enzymatic agent is selected from a group consisting of: acetic acid, pyruvic acid, phosphoric acid, salicylic acid, benzoic acid, urea and malic acid.

48. The method according to claim 38, wherein the composition comprises silver ions.

49. The method according to claim 48, wherein the composition further comprising at least one substance selected from the group consisting of: broad-spectrum antibiotics, chlorohexidine, povidone iodine, sulfamyalone, neosporin, metronidazole, chlorine dioxide (ClO₂) and polymyxin B sulfate.

50. The method according to claim 38, wherein the covering means is occlusive.

51. The method according to claim 38, wherein the covering means is water-impermeable.

52. The method according to a claim 38, wherein the covering means comprises a material selected from the group consisting of: gauze, absorbent pad, fibrous nets, fibrous knotted nets, non-woven dressing and sponges.

53. The method according to claim 38, wherein the debriding composition is provided in a storable non-active form, comprising reconstituting the debriding composition to obtain an active debriding composition prior to step (b).

54. The method according to claim 53, wherein the storable non-active debriding composition is reconstituted in a pharmaceutically acceptable carrier.

55. The method according to claim 54, wherein the pharmaceutically acceptable carrier is selected from the group consisting of: water, organic solvent, inorganic solvent, buffering agent, acidifying agent and alkalizing agent.

56. The method according to claim 54, wherein the pharmaceutically acceptable carrier is the antiseptic composition.

57. The method according to claim 53, wherein the storable non-active debriding composition is reconstituted no longer than 60 minutes before contacting the reconstituted debriding composition with the devitalized tissue.

58. The method according to claim 53, wherein the storable non-active debriding composition is in a form selected from the group consisting of: frozen, lyophilized, precipitated or crystallized.

59. The method according to claim 38 comprising contacting the skin with at least one analgesic agent, prior to step (a).

60. The method according to claim 59, wherein the analgesic agent is selected from the group consisting of: prilocaine, lidocaine, tetracaine, procaine, mepivacaine, benzocaine, bupivacaine, etidocaine and a combination thereof.
